# EUROPEAN PATENT APPLICATION

(11) **EP 3 851 119 A1**
(43) Date of publication of application: **21.07.2021**
(21) Application number: 21151027.6
(22) Date of filing: 14.08.2015
(51) Int. Cl.: A61K 38/46, A61K 9/00, A61P 17/00, A61P 31/14

(54) **TOPICAL COMPOSITION CONTAINING RANPIRNASE**

(30) Priority: 18.08.2014 US 201414462520; 08.07.2015 US 201514793920
(62) Divisional of application: 15754097.2
(71) Applicant: Orgenesis Inc., Germantown, Maryland 20876 (US)
(72) Inventor: SULLEY, Jamie, La Jolla, CA California 92037 (US); SQUIQUERA, Luis, 1425 Buenos Aires (AR)
(74) Representative: Pearl Cohen Zedek Latzer Baratz UK LLP

(57) **Abstract**

A topical composition comprising a therapeutically effective quantity of an enzymatically-active ribonuclease as the sole active agent for use in the treatment of a skin disease in a patient by topically applying the composition to the affected areas of the skin.

SEQUENCE LISTING

<110>  Orgenesis, Inc.

<120>  Topical Composition Containing Ranpirnase

<130>  P-595208-EP1

<150>  EP 15754097.2
<151>  2015-08-14

<150>  US 14/462,520
<151>  2014-08-18

<150>  US 14/793,920
<151>  2015-07-08

<160>  4

<170>  PatentIn version 3.5

<210>  1
<211>  104
<212>  PRT
<213>  Rana pipiens

<400>  1

Glu Asp Trp Leu Thr Phe Gln Lys Lys His Ile Thr Asn Thr Arg Asp
1               5                   10                  15

Val Asp Cys Asp Asn Ile Met Ser Thr Asn Leu Phe His Cys Lys Asp
            20                  25                  30

Lys Asn Thr Phe Ile Tyr Ser Arg Pro Glu Pro Val Lys Ala Ile Cys
        35                  40                  45

Lys Gly Ile Ile Ala Ser Lys Asn Val Leu Thr Thr Ser Glu Phe Tyr
    50                  55                  60

Leu Ser Asp Cys Asn Val Thr Ser Arg Pro Cys Lys Tyr Lys Leu Lys
65                  70                  75                  80

Lys Ser Thr Asn Lys Phe Cys Val Thr Cys Glu Asn Gln Ala Pro Val
                85                  90                  95

His Phe Val Gly Val Gly Ser Cys
            100

<210>  2
<211>  104
<212>  PRT
<213>  artificial

<220>
<223>  '805 variant of ranpirnase

<400>  2

Glu Asp Trp Leu Thr Phe Gln Lys Lys His Val Thr Asn Thr Arg Asp
1               5                   10                  15

Val Asp Cys Asn Asn Ile Met Ser Thr Asn Leu Phe His Cys Lys Asp
            20                  25                  30

Lys Asn Thr Phe Ile Tyr Ser Arg Pro Glu Pro Val Lys Ala Ile Cys
        35                  40                  45

Lys Gly Ile Ile Ala Ser Lys Asn Val Leu Thr Thr Ser Glu Phe Tyr
    50                  55                  60

Leu Ser Asp Cys Asn Val Thr Ser Arg Pro Cys Lys Tyr Lys Leu Lys
65                  70                  75                  80

Lys Ser Thr Asn Lys Phe Cys Val Thr Cys Glu Asn Gln Ala Pro Val
                85                  90                  95

His Phe Val Gly Val Gly Arg Cys
            100


<210>  3
<211>  114
<212>  PRT
<213>  Rana pipiens

<400>  3

Lys Pro Lys Glu Asp Arg Glu Trp Glu Lys Phe Lys Thr Lys His Ile
1               5                   10                  15

Thr Ser Gln Ser Val Ala Asp Phe Asn Cys Asn Arg Thr Met Asn Asp
            20                  25                  30

Pro Ala Tyr Thr Pro Asp Gly Gln Cys Lys Pro Ile Asn Thr Phe Ile
        35                  40                  45

His Ser Thr Thr Gly Pro Val Lys Glu Ile Cys Arg Arg Ala Thr Gly
    50                  55                  60

Arg Val Asn Lys Ser Ser Thr Gln Gln Phe Thr Leu Thr Thr Cys Lys
65                  70                  75                  80

Asn Pro Ile Arg Cys Lys Tyr Ser Gln Ser Asn Thr Thr Asn Phe Ile
                85                  90                  95

Cys Ile Thr Cys Arg Asp Asn Tyr Pro Val His Phe Val Lys Thr Gly

            100                 105                 110

Lys Cys

<210> 4
<211> 115
<212> PRT
<213> artificial

<220>
<223> rAmphinase 2

<400> 4

Met Lys Pro Lys Glu Asp Arg Glu Trp Glu Lys Phe Lys Thr Lys His
1               5                   10                  15

Ile Thr Ser Gln Ser Val Ala Asp Phe Asn Cys Asn Arg Thr Met Asn
            20                  25                  30

Asp Pro Ala Tyr Thr Pro Asp Gly Gln Cys Lys Pro Ile Asn Thr Phe
        35                  40                  45

Ile His Ser Thr Thr Gly Pro Val Lys Glu Ile Cys Arg Arg Ala Thr
    50                  55                  60

Gly Arg Val Asn Lys Ser Ser Thr Gln Gln Phe Thr Leu Thr Thr Cys
65                  70                  75                  80

Lys Asn Pro Ile Arg Cys Lys Tyr Ser Gln Ser Asn Thr Thr Asn Phe
                85                  90                  95

Ile Cys Ile Thr Cys Arg Asp Asn Tyr Pro Val His Phe Val Lys Thr
            100                 105                 110

Gly Lys Cys
        115

## Description

### Summary of the Invention

The invention relates to antiviral pharmaceuticals, and more particularly relates to antiviral pharmaceuticals for topical administration. In its most immediate sense, the invention relates to pharmaceuticals for treating patients with anogenital warts.

### Background of the Invention

Ranpirnase is a protein with ribonuclease activity, it has a molecular weight of approximately 12,000 Daltons, and it has an amino acid sequence disclosed and claimed in U.S. Patent No. 5,559,212. It can be isolated from embryos and eggs of the *Rana pipiens* frog or produced as a recombinant protein (see e.g. Patent No. US 6,175,003 B1). Commonly-owned patent number US 8,663,964 B2 teaches that ranpirnase and another enzymatically active ribonuclease are active against human papillomavirus (hereinafter, "HPV") and that HPV can be treated by using either of these two ribonucleases on an HPV-infected region of a patient. Anogenital warts are caused by various human papillomaviruses, and no satisfactory treatment exists for this sexually transmitted disease since all available treatment modalities target the lesions and lack viricidal activity against HPV.

It has been proposed to treat anogenital warts intralesionally, i.e. by injecting an active pharmaceutical ingredient ("API") into the wart to be treated. In many situations - where the warts are small or too numerous, or in locations where an injection would be too painful - this would be unsatisfactory. It has also been proposed to treat anogenital warts topically. Because the HPV-infected cells are located beneath the surface of the patient's skin and would not be directly contacted by the API, this proposal assumed that it would be necessary to administer the API using a special vehicle that would penetrate through the intervening layers of the patient's skin to thereby deliver the API to a location where its anti-HPV activity would be useful.

### Summary of the Invention

The present invention relates to a pharmaceutical composition for topical administration comprising a therapeutically effective quantity of an enzymatically-active ribonuclease and a vehicle that does not unacceptably interfere with such enzymatic activity.

Preferably, the enzymatically-active ribonuclease has an amino acid sequence selected from the group consisting of SEQ ID Nos. 1, 2, 3 and 4 and more preferably the enzymatically-active ribonuclease is ranpirnase.

Also preferably, the vehicle is an aqueous vehicle and more preferably the vehicle is a gel, a serum, a spray or a lotion.

In a preferred embodiment the vehicle comprises glycerol and/or a cellulose derivative. In another embodiment the vehicle comprises an ethoxylated lipid and, optionally, an alcohol and/or an aqueous adjuvant such as *Aloe vera.*

In another preferred embodiment the vehicle is an oil-in-water solution and more preferably the vehicle is a cream, emulsion, ointment or a micellar system.

Also preferably, the pharmaceutical composition contains between 0.0.1 and 5 mg of the ribonuclease per ml of vehicle.

Also preferably, the pharmaceutical composition is to be administered one to five times per day.

In a further preferred embodiment the concentration of ribonuclease in the pharmaceutical is chosen to deliver between 1 and 3 mg of ribonuclease to the patient each week that the pharmaceutical is administered to the patient in accordance with the dosage regimen used.

The present invention also relates to said pharmaceutical composition for use in treating a virus-mediated disease, preferably a disease which is mediated by human papilloma virus and most preferably a disease which is mediated by HPV11.

In a preferred embodiment the disease are warts, more preferably anogenital warts.

The present invention also relates to said pharmaceutical composition for use in treating a skin disease such as psoriasis and actinic keratosis.

### Brief Description of the Drawings

Fig. 1 shows the global performance of evaluable patients treated with the invention in a Phase I study.
Fig. 2 shows the number of patients who achieved clinical healing at weeks 4 and 8 in the Phase I study.
Fig. 3 shows the time required for patients in the Phase I study to reach clinical healing.

### Detailed Description of Preferred Embodiments

A Phase I compassionate use observational study was carried out in Argentina on male volunteers with anogenital warts. Commonly-owned parent patent application 14/462,520 filed August 18, 2014 discloses an unexpected and surprising result from this study, namely, that it is unnecessary to administer the API using a vehicle that would penetrate through the layers of the patient's skin. Rather, that study demonstrated that topical ranpirnase therapy for HPV did not - as was expected - require a vehicle having penetrating characteristics. Rather, it appeared that the vehicle need only not unacceptably interfere with the enzymatic activity of ranpirnase. Furthermore, because there are other enzymatically-active ribonucleases that behave similarly to ranpirnase, the referenced parent patent application stated that it was reasonable to expect that any such ribonuclease would, when combined with a suitable vehicle, have an activity similar to that of ranpirnase.

This Phase I study has now been completed, with most favorable results. While the study lasted for only eight weeks, in more than 80% of the evaluable patients no warts were visible, i.e. those patients appeared to be "clinically healed". To a person of ordinary skill in the art, this is strong evidence that the invention is reasonably correlated with usefulness in treating HPV. Significantly, it is believed that the vehicle need not be entirely free of anti-enzymatic activity. In some instances, it is believed possible to overcome anti-enzymatic qualities of the carrier by increasing the concentration of the enzymatically-active ribonuclease.

In accordance with the invention, a pharmaceutical comprises a therapeutically effective quantity of an enzymatically-active ribonuclease (Rnase) and a vehicle that does not unacceptably interfere with such enzymatic activity. Preferably, the RNase is from the RNase A superfamily.

The pharmaceutical composition is intended to be administered topically, i.e. to a surface of the patient's body such as the skin or mucous membranes. Preferably, the pharmaceutical composition is administered to the patient's skin such as in the treatment of anogenital warts. The topical administration according to the present invention also includes vaginal, extra-vaginal (outside of the vagina), intra-vaginal (inside the vagina), anal, peri-anal (surrounding the anus) and intra-anal (inside the anus) administration.

RNases of the RNase A superfamily are pyrimidine-specific endonucleases found in high quantity in the pancreas of certain mammals and of some reptiles. They are involved in endonucleolytic cleavage of 3'-phosphomononucleotides and 3'-phosphooligonucleotides ending in C-P or U-P with 2',3'-cyclic phosphate intermediates. Members of this superfamily include ranpirnase and variants thereof, amphinase, r-Amphinase-2, bovine seminal vesicle and brain ribonucleases; kidney non-secretory ribonucleases; liver-type ribonucleases, angiogenin; eosinophil cationic protein, and pancreatic ribonucleases from different species including human and bovine pancreatic ribonucleases.

Ranpirnase is an RNase isolated from oocytes of the leopard frog *Rana pipiens* which is disclosed in U.S. Pat. No. 5,559,212, and is also known as Onconase®. The amino acid sequence of ranpirnase is provided in SEQ ID NO: 1. Ranpirnase has been tested and found to be cytotoxic to cancer cells because of its enzymatic activity against RNA.

A variant of ranpirnase is disclosed in U.S. Pat. No. 5,728,805 (hereinafter, the "'805 variant"). The '805 variant is also an RNase, and has likewise been found to be cytotoxic to certain cancer cells. The '805 variant is a close variant of ranpirnase; its amino acid sequence is identical to that of ranpirnase except that it has valine instead of isoleucine at position 11, asparagine instead of aspartic acid at position 20, and arginine instead of serine at position 103 of the ranpirnase amino acid sequence. In some embodiments, the '805 variant is referred to as "Val11, Asn20, Arg103-Ranpirnase". The amino acid sequence of the '805 variant is provided in SEQ ID NO:2.

Amphinase 2 is also an RNase. It is the protein identified as 2325p4 in U.S. Pat. No. 6,239,257 and it too has been found to be cytotoxic to cancer cells. The amino acid sequence of Amphinase 2 is provided in SEQ ID NO: 3.

Recombinant Amphinase 2 ("rAmphinase 2") is similar to Amphinase 2, but has a Met residue at position -1 and lacks glycan moieties that are located in Amphinase 2 at positions 27 and 91. rAmphinase 2 is described in U.S. Pat. No. 7,229,824. The amino acid sequence of rAmphinase 2 is provided in SEQ ID NO: 4.

The term "functionally equivalent thereof" is intended to comprise proteins which differ from naturally occurring RNases by one or more amino acids, but retain RNase activity. For example, the '805 variant discussed above may be considered as a functional equivalent of ranpirnase, since it differs from ranpirnase in some amino acids, but retains RNase activity.

U.S. Pat. Nos. 5,559,212, 5,728,805, 6,239,257, 7,229,824, 8,518,399, 8,663,964, and U.S. Publication Nos. 2012-0003266 and 2014-0037610 are all incorporated by reference herein in their entireties for all purposes.

Ranpirnase is known to degrade tRNA very effectively (see Lin et al., Biochemical and Biophysical Research Communications 201 (1), 156 - 162 (1994)). Because normal mammalian cells degrade approximately 80% of their tRNA as a natural process, this degradation causes little if any harm to the cells themselves. As a result, except in instances where a viral infection has spread too far to be effectively controlled, a systemic application of ranpirnase, particularly before the viral infection has spread too far, causes the virus to die out without killing the normal cells that the virus infects.

Preferred embodiments of the invention use an RNase according to any of SEQ ID Nos. 1 to 4 as the enzymatically-active ribonuclease. More preferably, the RNase has the sequence according to SEQ ID No. 1 or 2 and most preferably the RNase is ranpirnase which has the sequence according to SEQ ID No. 1.

In one embodiment of the present invention the vehicle is an aqueous vehicle.

The term "aqueous vehicle" is intended to mean that the basis of the pharmaceutical composition is formed by water which is typically mixed with one or more other water-soluble ingredients. Accordingly, the pharmaceutical composition based on an aqueous vehicle is water-soluble.

Preferred aqueous vehicles are liquids, gels, lotions, or approved sexual lubricants (which may themselves be gels or lotions). This is because gels, lotions, and sexual lubricants are viscous or can be made viscous so that the invention will remain where it has been applied and will not run off. The viscosity of any of the above vehicles should be such that it remains where it has been applied. The mentioned vehicles can be used with sprays, dropper, brush or roll on applicators to ease the contact between the formulation and the target lesion.

The term "gel" is intended to include homogeneous, clear, semi-solid preparations consisting of a liquid phase within a three-dimensional polymeric matrix with physical or sometimes chemical cross-linkage by means of suitable gelling agents.

The term "lotion" is intended to comprise an emulsion liquid dosage form for external application to the skin which usually contains an aqueous vehicle and >50% water and volatiles.

A "spray" is a pressurized dosage form containing a therapeutic agent which upon actuation of the device can emit a fine dispersion of liquid and/or solid materials in a gaseous medium.

"Sexual lubricants" are usually used during human sexual acts to reduce the friction between the genital parts or in medicine to reduce the friction between medical instruments and genital parts. Known aqueous-based sexual lubricants include Astroglide, Elbow Grease, Good Clean Love, Gynol II, ID Glide Ultra, KY Jelly, PRE, Replens, Slippery Stuf fand Sliquid Organic.

Preferably, the sexual lubricant contains glycerol and/or a cellulose derivative as a lubricant. The cellulose derivative may include hydroxyethyl cellulose, sodium carboxymethyl cellulose and/or cellulose polymer. Preferably, the cellulose derivative is hydroxyethyl cellulose.

A particularly preferred sexual lubricant contains chlorhexidine gluconate, gluconolactone, glycerol, hydroxyethyl cellulose, methylparaben and sodium hydroxide. Such a sexual lubricant is known as KY Jelly.

Alternatively, the vehicle may be an oil-in-water solution meaning that the vehicle comprises an oil phase and a water phase which are not miscible with each other. Suitable oil in water solutions include creams, emulsions, ointments and micellar systems.

The term "ointment" is intended to include homogeneous, semi-solid preparations intended for external application to the skin or mucous membranes. It usually contains <20% water and volatiles and >50% of hydrocarbons, waxes, or polyethylene glycols as the vehicle for external application to the skin.

The term "cream" is intended to include homogeneous, semi-solid preparations consisting of opaque emulsion systems.

An emulsion is a mixture of two liquids which are not miscible such as an oil phase and a water phase.

"Micellar systems" are formed by amphiphilic compounds, wherein the hydrophilic part is in contact with a hydrophilic liquid, whereas the hydrophobic part is within the micelle. Micelles may incorporate a pharmaceutical compound within their interior.

In another preferred embodiment a transdermal delivery system is used which comprises an ethoxylated lipid such as ethoxylated castor oil or ethoxylated macadamia nut oil, optionally mixed with an alcohol such as isopropyl alcohol, as penetration enhancer and *Aloe vera* as an aqueous adjuvant. Methods for preparing such a delivery system are described in U.S. Patents Nos. 6,759,056, 6,946,144, 7,201,919, 7,220,427, 7,300,666, and 7,316,820.

Advantageously, and in accordance with preferred embodiments of the invention, the pharmaceutical composition has between 0.0.1 and 5 mg or 0.02 to 4.5 mg of the enzymatically active ribonuclease per ml of vehicle, more preferably , the pharmaceutical composition has between 0.1 and 4 mg or 0.5 to 3.5 mg of the enzymatically active ribonuclease per ml of vehicle and even more preferably, the pharmaceutical composition has between 1 and 3 mg of the enzymatically active ribonuclease per ml of vehicle. Most preferably, the pharmaceutical composition has 1 mg of the enzymatically active ribonuclease per ml of vehicle.

The pharmaceutical composition can be prepared by mixing a solution of the ribonuclease or the ribonuclease in lyophilized form in the required amount with the vehicle. Such composition could be packed in two separate vials and provided and mixed before use. The final solution should be kept refrigerated in order to avoid bacterial contamination. Ranpirnase is resistant to heat and can be stored at room temperature as well of high concentration of caotropic agents which assures its stability in different media.

The pharmaceutical composition is administered one to five times per day, preferably two or three times per day. If the pharmaceutical composition uses a sexual lubricant such as KY Jelly as vehicle, the pharmaceutical composition is preferably applied three times per day. If the pharmaceutical composition uses the polysaccharide formulation of JRX Biotechnology, the pharmaceutical composition is preferably applied two times per day.

The pharmaceutical composition may also be delivered continuously, meaning that the pharmaceutical composition is applied to the skin, e.g. in form of a patch or a soft gel, and the RNase is released continuously to the skin over a certain period.

The pharmaceutical composition of the present invention is preferably used to treat a virus-mediated disease.

The virus-mediated disease may be any disease which is caused by a virus and which can be treated by topical administration of a pharmaceutical composition. Such virus-mediated diseases include infections with Herpes simplex virus, Epstein-Barr-Virus and or Herpes zoster, warts and Molluscum contagiosum.

A Herpes simplex virus infection is caused by herpes simplex virus-1 (HSV-1) or herpes simplex virus-2 (HSV-2) which belong to the family of herpesviridae and are linear double-stranded DNA viruses.

A Herpes zoster infection is caused by the varizella zoster virus (VZV) which belongs to the family of herpesviridae and is a linear double-stranded DNA virus.

Epstein-Barr virus which also belongs to the family of herpesviridae and has a linear double-stranded DNA genome causes hairy leukoplakia which is a white patch on the side of the tongue with a corrugated or hairy appearance.

Warts are common and benign epithelial growths caused by human papillomavirus (HPV) which belongs to the family of papillomaviridae and is a circular double-stranded DNA virus.

Molluscum contagiosum is a viral infection of the skin or occasionally of the mucous membranes, sometimes called water warts. It is caused by a DNA poxvirus called the *molluscum contagiosum virus* (MCV) which belongs to the family of poxviridae and is a linear double-stranded DNA virus.

Preferably, the virus-mediated targeted disease are warts which are caused by human papillomavirus. More preferably, the warts are anogenital warts, i.e. warts located at the anus and/or the genital tract. Also preferably, the warts are caused by human papillomavirus type 11.

The pharmaceutical composition of the present invention can also be used to treat skin diseases which are not caused by virus infections, in particular those skin diseases which are associated with increased cell proliferation. Such skin diseases include different types of skin cancer such as melanoma, basal-cell carcinoma and squamous cell carcinoma and preceding stages thereof such as actinic keratosis, but also autoimmune diseases such as psoriasis.

The patient is treated with the pharmaceutical composition of the present invention for at least two weeks, preferably for at least four weeks and most preferably for eight weeks.

The treatment with the pharmaceutical composition of the present invention results in clinical healing in at least 20% of the patients treated for four weeks and in clinical healing in at least 70%, preferably 80% and more preferably 90% of the patients treated after treatment for eight weeks. The term "clinical healing" means that no visible signs of the viral disease are visible. In the case of warts such as warts caused by human papillomavirus, the term "clinical healing" means that no warts are detectable after treatment with the pharmaceutical composition of the present invention.

### Example

A Phase I compassionate use clinical study was conducted in Buenos Aires. Male volunteers who were diagnosed with anogenital warts in various locations (scrotum, penis shaft, penis dorsum, inguinal, perianal) were accrued to the study. The study had three arms. In arm A, the tested embodiment was ranpirnase combined with a vehicle supplied by JRX Biotechnology, Inc. (In this instance, the ranpirnase was reconstituted from lyophilized powder.) In arm C, the tested embodiment was also ranpirnase combined with the same JRX Biotechnology, Inc. vehicle, but in this instance the ranpirnase had been previously frozen and was thawed prior to use. These two combinations were very low-viscosity liquids; each was applied topically twice each day. The vehicle used in these two arms is a polysaccharide formulation; on information and belief it is covered by U.S. Patents Nos. 6,759,056, 6,946,144, 7,201,919, 7,220,427, 7,300,666, and 7,316,820.

In arm B, the tested embodiment was ranpirnase combined with K-Y® Brand Jelly. This second embodiment was formulated from ranpirnase reconstituted from lyophilized powder; it is a viscous gel that was applied topically three times each day.

In each arm, the concentration of ranpirnase was 1 mg ranpirnase to 1 ml of vehicle.

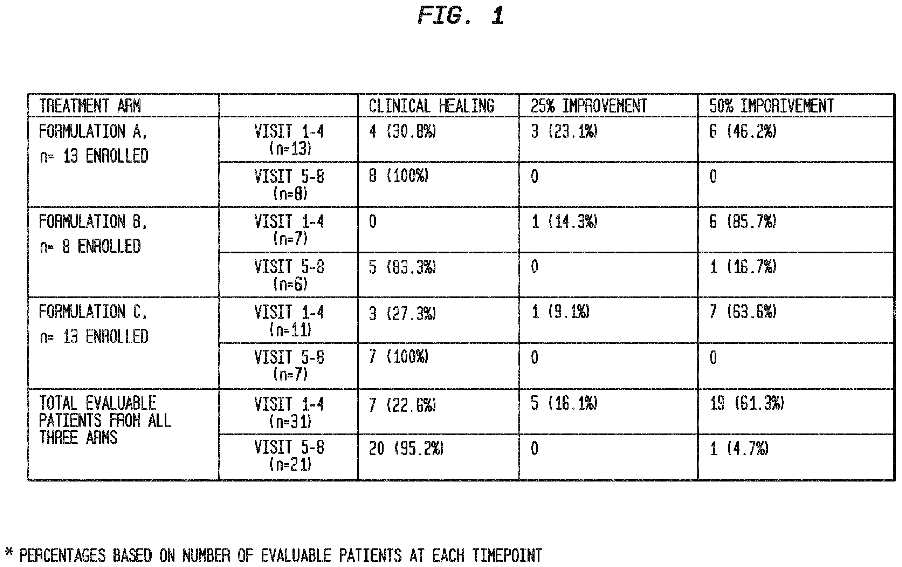

As can be seen in Fig. 1, 22.6% of all the patients in the study achieved clinical healing by the 4th week of treatment, with 61.3% of all the patients having reached a 50% improvement by that time. Fig. 1 also shows that 95.2% of all the patients achieved clinical healing by the 8th week and another 4.7% of all the patients at least reached a 50% improvement in this time. Although the long-term effect of this treatment is not yet known, this shortterm effect is far better than has been reported for other pharmaceuticals (Podofilox solution and gel, Aldara Cream, Veregen ointment).

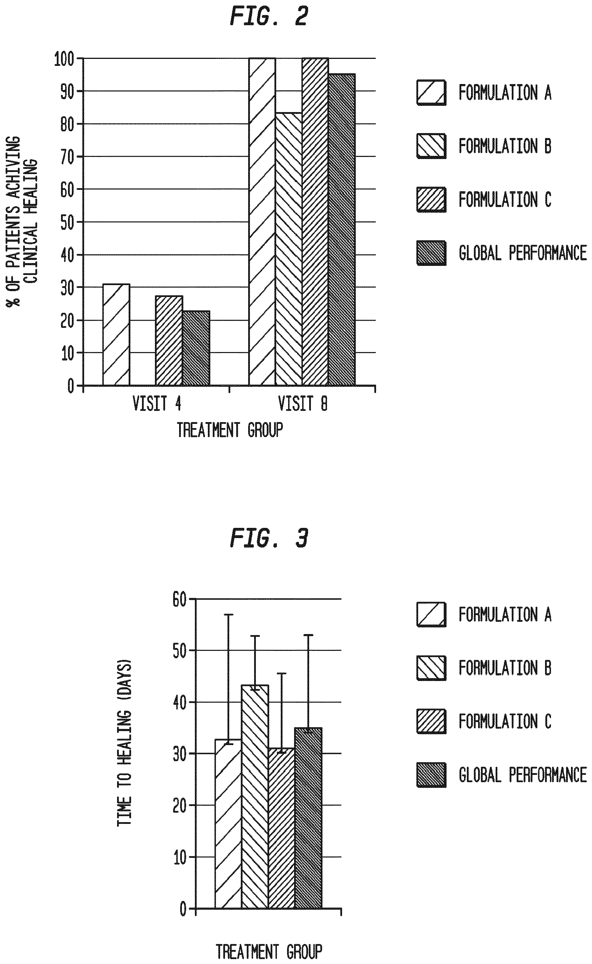

Fig. 2 graphically displays the information in the three leftmost columns in Fig. 1. To a person of ordinary skill in this art, Fig. 2 is strong evidence that the invention is reasonably correlated with usefulness in treating HPV. Fig. 2 shows that 100% of the evaluable patients in arms A and C achieved clinical healing by the 8th week, and 83.3% of the patients in arm B achieved clinical healing by that time. Overall, 95.2% of all patients achieved clinical healing by the 8th week.

Fig. 3 displays the average time required for patients in each of the three arms, and in the study as a whole, to reach clinical healing. The patients in arm B required more than 40 days to reach clinical healing, while the patients in arms A and C reached clinical healing sooner (in approximately 32 and 34 days, respectively).

This study did not address the question of dosage; in all arms of the study the quantity of ranpirnase delivered to the patient was 1 mg/ week. The activity of the preferred embodiment may be improved by increasing the concentration of ranpirnase from 1 mg/ml to 3 mg/ml, whereby the weekly dose of ranpirnase administered to each patient would be increased to 3 mg, but this has not yet been tested.

It will be understood that although the invention has been developed for use in treatment of anogenital warts, its use is not restricted to this application. The invention may have other antiviral applications. Although the invention is presently applied to external anogenital warts, it may also be useful when applied vaginally, extra-vaginally, intra-vaginally, anally, peri-anally, and intra-anally. Although the preferred embodiment uses ranpirnase as the enzymatically-active ribonuclease, other ribonucleases having similar enzymatic activities exist and may be used instead. Furthermore, while treatment of anogenital warts may be easier using a viscous vehicle such as a gel because it is easier to apply a gel to an anogenital wart and a gel is less likely to run off and is therefore more likely to remain where it has been applied, there may be other applications in which a liquid vehicle, or an ointment vehicle will be preferable. The vehicle may alternatively be a serum, a lotion, or an approved sexual lubricant, i.e. a lubricant that meets applicable governmental requirements and is intended for use on human genitalia.

Although at least one preferred embodiment of the invention has been described above, this description is not limiting and is only exemplary. The scope of the invention is defined only by the claims, which follow.

Embodiments of the invention, corresponding to the original claims of PCT/US2015/045272 (published as WO 2016/028634), include the following:
1. A pharmaceutical composition for topical administration comprising a therapeutically effective quantity of an enzymatically-active ribonuclease and a vehicle that does not unacceptably interfere with such enzymatic activity.
2. The pharmaceutical composition of embodiment 1, wherein the enzymatically-active ribonuclease has an amino acid sequence selected from the group consisting of SEQ ID Nos. 1, 2, 3 and 4.
3. The pharmaceutical composition of embodiment 1, wherein the enzymatically-active ribonuclease is ranpirnase.
4. The pharmaceutical composition of any one of embodiments 1 to 3, wherein the vehicle is an aqueous vehicle.
5. The pharmaceutical composition of embodiment 4, wherein the vehicle is a gel or a lotion.
6. The pharmaceutical composition of any one of embodiments 1 to 3, wherein the vehicle is an oil-in-water solution.
7. The pharmaceutical composition of embodiment 6, wherein the vehicle is a cream, emulsion, ointment or a micellar system.
8. The pharmaceutical composition of any one of embodiments 1 to 7, wherein the vehicle comprises glycerol.
9. The pharmaceutical composition of any one of embodiments 1 to 8, wherein the vehicle comprises a cellulose derivative.
10. The pharmaceutical composition of any one of embodiments 1 to 7, wherein the vehicle comprises an ethoxylated lipid.
11. The pharmaceutical composition of any one of embodiments 1 to 10, wherein the pharmaceutical composition contains between 0.01 and 5 mg of the ribonuclease per ml of vehicle.
12. The pharmaceutical composition of any one of embodiments 1 to 11, wherein the pharmaceutical composition is to be administered one to five times per day.
13. The pharmaceutical composition of any one of embodiments 1 to 12, wherein the concentration of ribonuclease in the pharmaceutical is chosen to deliver between 1 and 3 mg of ribonuclease to the patient each week that the pharmaceutical is administered to the patient in accordance with the dosage regimen used.
14. Pharmaceutical composition of any of embodiments 1 to 13 for use in treating a virus-mediated disease.
15. Pharmaceutical composition for use according to embodiment 14, wherein the disease is mediated by human papilloma virus.
16. Pharmaceutical composition for use according to embodiment 15, wherein the human papilloma virus is HPV11.
17. Pharmaceutical composition for use according to any one of embodiments 14 to 16, wherein the disease is warts.
18. Pharmaceutical composition of any one of embodiments 1 to 13 for use in treating a skin disease.

## Claims

1. A topical composition comprising a therapeutically effective quantity of an enzymatically-active ribonuclease as a sole active agent, and an aqueous, viscous vehicle that does not unacceptably interfere with the enzymatic activity of the ribonuclease, wherein the aqueous, viscous vehicle is a gel, a lotion, cream, emulsion, ointment or a micellar system, for use in the treatment of a skin disease in a patient, wherein the treatment comprises topically applying the composition to the affected skin areas of the patient, wherein the skin disease is selected from skin diseases which are not caused by virus infections, skin diseases associated with increased cell proliferation, skin cancer, psoriasis, and actinic keratosis.

2. A topical composition comprising a therapeutically effective quantity of an enzymatically-active ribonuclease as a sole active agent, and an aqueous, viscous vehicle that does not unacceptably interfere with the enzymatic activity of the ribonuclease, wherein the aqueous, viscous vehicle is a gel, a lotion, cream, emulsion, ointment or a micellar system, for use in the treatment of a Herpesviridae infection in a patient, wherein the treatment comprises topically applying the composition to the affected skin areas of the patient.

3. The topical composition for use of claim 2, wherein the Herpesviridae infection is selected from herpes simplex virus (HSV), herpes simplex virus- 1 (HSV-1), herpes simplex virus-2 (HSV-2), Epstein-Barr-virus (HBV), herpes zoster virus (HZV), and varicella zoster virus (VZV).

4. A topical composition comprising a therapeutically effective quantity of an enzymatically-active ribonuclease as a sole active agent, and an aqueous, viscous vehicle that does not unacceptably interfere with the enzymatic activity of the ribonuclease, wherein the aqueous, viscous vehicle is a gel, a lotion, cream, emulsion, ointment or a micellar system, for use in the treatment of a Molluscum Contagiosum Virus (MCV) infection in a patient, wherein the treatment comprises topically applying the composition to the affected skin areas of the patient.

5. A topical composition comprising a therapeutically effective quantity of an enzymatically-active ribonuclease as a sole active agent, and an aqueous, viscous vehicle that does not unacceptably interfere with the enzymatic activity of the ribonuclease, wherein the aqueous, viscous vehicle is a gel, a lotion, cream, emulsion, ointment or a micellar system, for use in the treatment of a cancer in a patient, wherein the treatment comprises topically applying the composition to the affected skin areas of the patient.

6. The topical composition for use according to claim 5, wherein the cancer is selected from melanoma, basal-cell carcinoma and squamous cell carcinoma.

7. The topical composition for use according to any of claims 1 to 6, wherein the enzymatically-active ribonuclease has an amino acid sequence selected from SEQ ID NOs. 1, 2, 3 and 4.

8. The topical composition for use according to any of claims 1 to 6, wherein the enzymatically-active ribonuclease is ranpirnase.

9. The topical composition for use according to any of claims 1 to 8, wherein the vehicle comprises glycerol.

10. The topical composition for use according to any of claims 1 to 9, wherein the vehicle comprises a cellulose derivative.

11. The topical composition for use according to any of claims 1 to 10, wherein the topical composition contains between 0.01 and 5 mg of the ribonuclease per ml of vehicle.

12. The topical composition for use according to any of claims 1 to 11, wherein the composition is administered one to five times per day.

13. The topical composition for use according to any of claims 1 to 12, wherein the therapeutically effective quantity of the ribonuclease delivers between 1 and 3 mg of ribonuclease to a patient each week that the topical composition is administered to the patient.

14. The topical composition for use according to any of claims 1 to 13, wherein the aqueous, viscous vehicle is a gel.

15. The topical composition for use according to any of claims 1 to 14, consisting of a therapeutically effective quantity of an enzymatically-active ribonuclease, and an aqueous, viscous vehicle that does not unacceptably interfere with the enzymatic activity of the ribonuclease, wherein the aqueous, viscous vehicle is a gel.
